# EUROPEAN PATENT SPECIFICATION

(11) **EP 2 282 722 B1**
(45) Date of publication and mention of the grant of the patent: **17.05.2017**
(21) Application number: 09734176.2
(22) Date of filing: 21.04.2009
(51) Int. Cl.: A61K 9/107, A61K 9/16, A61K 9/50

(54) **SELF-MICROEMULSIFYING SYSTEMS INCORPORATED INTO LIQUID CORE MICROCAPSULES**
SELBSTMIKROEMULGIERENDE SYSTEME IN MIKROKAPSELN MIT FLÜSSIGKERN
SYSTÈME À MICRO-ÉMULSIFICATION AUTOMATIQUE INTÉGRÉ DANS DES MICROCAPSULES À COEUR LIQUIDE

(30) Priority: 22.04.2008 EP 08154947
(43) Date of publication of application: 16.02.2011
(73) Proprietor: LEK Pharmaceuticals d.d., 1526 Ljubljana (SI)
(72) Inventor: KERC, Janez, 1526 Ljubljana (SI); HOMAR, Miha, 1526 Ljubljana (SI); GASPERLIN, Mirjana, 1000 Ljubljana (SI)
(74) Representative: TBK
(86) International application number: PCT/EP2009/054756
(87) International publication number: WO 2009/130225

(56) References cited:
- US-A- 5 753 264
- RIBEIRO A J ET AL: "MICROENCAPSULATION OF LIPOPHILIC DRUGS IN CHITOSAN-COATED ALGINATE MICROSPHERES" INTERNATIONAL JOURNAL OF PHARMACEUTICS, ELSEVIER BV, NL, vol. 187, 30 September 1999 (1999-09-30), pages 115-123, XP001120054 ISSN: 0378-5173 cited in the application
- HOMAR MIHA ET AL: "Preparation of microcapsules with self-microemulsifying core by a vibrating nozzle method" JOURNAL OF MICROENCAPSULATION, TAYLOR AND FRANCIS, BASINGSTOKE, GB, vol. 24, no. 1, 1 February 2007 (2007-02-01), pages 72-81, XP007905692 ISSN: 0265-2048 cited in the application
- KIM C-K ET AL: "ONCE-A-DAY ORAL DOSING REGIMEN OF CYCLOSPORIN A: COMBINED THERAPY OF CYCLOSPORIN A PREMICROEMULSION CONCENTRATES AND ENTERIC COATED SOLID-STATE PREMICROEMULSIONS CONCENTRATES" PHARMACEUTICAL RESEARCH, NEW YORK, NY, US, vol. 18, no. 4, 1 April 2001 (2001-04-01), pages 454-459, XP001030025 ISSN: 0724-8741 cited in the application

## Description

The present invention relates to the field of microemulsions or self-microemulsifying systems (SMES), and microcapsules allowing the formulation of microemulsions or self-microemulsifying systems into solid dosage forms. Specifically, the invention discloses microcapsules with a liquid core incorporating a self-microemulsifying system or a microemulsion, a method for producing such microcapsules, and pharmaceutical formulations comprising such microcapsules.

The increasing problem of new active ingredients with poor water solubility is a well known fact in the pharmaceutical industry. Application of such drugs in lipid vehicles can greatly increase their bioavailability. However, use of pure lipid carriers is not suitable for most drugs, although special substances, such as vitamins A and D, have been delivered in this way for a long time. The formulation of emulsions, microemulsions and other similar systems with lipids can greatly enhance bioavailability of many substances.

Emulsions are mixtures of two immiscible liquids phases, e.g. oil and water. They are formed by high energy input, which is required to disperse one phase in the form of droplets into another phase. These systems are usually very unstable and quickly both liquid phases separate. To increase the stability of these systems various additives can be used, e.g. surfactants. Nevertheless, even with various additives, all emulsion are thermodynamically unstable and the separation of the mixed phases is inevitable. Emulsions tend to have a cloudy appearance due to large droplet size of the dispersed phase, which scatter the light that passes through the emulsion.

In contrast, microemulsions are clear, stable, isotropic liquid mixtures of lipophilic phase, water and one or more surface active substances (surfactant, co-surfactant). The aqueous phase may contain salt(s) and/or other soluble ingredients. Additionally, a cosolvent can also be included in the microemulsions in order to increase the solubility of further ingredients of the microemulsions, such as, for example, active pharmaceutical ingredients. In contrast to ordinary emulsions, microemulsions form upon simple mixing of the components and do not require the high shear conditions generally used in the formation of ordinary emulsions. Microemulsions have many advantages over emulsions, including thermodynamic stability, greater drug solubilization capacity and permeability enhancement. Because their droplet size is smaller than that of emulsions, they are usually transparent and have a greater specific surface area resulting in faster drug dissolution from microemulsions in comparison to emulsions. In addition, the intra- and inter-patient variability of pharmacokinetic parameters is reduced when a drug is administered in form of a microemulsion.

Self-microemulsifying systems (SMES) are systems consisting of a mixture of an oily (lipophilic) phase comprising at least one lipophilic substance with one or more surface active substance (surfactant, co-surfactant), which spontaneously form microemulsions upon contact with aqueous media. Additionally, a cosolvent can also be included in the SMES in order to increase the solubility of further ingredients of the SMES, such as, for example, active pharmaceutical ingredients. Compared to microemulsions, SMES do not contain water and thus exhibit a better physical and microbiological stability. They share all the advantages of microemulsions over coarse emulsions, including thermodynamic stability, greater drug solubilization capacity and permeability enhancement. Incorporation of drugs in self-microemulsifying systems thus offers several advantages for their delivery, the main one being faster drug dissolution and absorption.

Microparticulate drug delivery systems, such as microcapsules and microspheres (or pellets), are widely used in pharmacy for a number of applications such as controlled oral delivery. Their advantages over single unit drug delivery systems are the absence of dose dumping and a larger surface area. The use of biodegradable substances, such as alginate and chitosan, has further benefits for safety. Microcapsules are micrometer-sized particles (10-2000 µm), outwardly similar to microspheres, but with a distinguishable core and shell. The active ingredient is generally located in the core and the shell is usually formed from polymeric material. The preparation of microcapsules with liquid core is seldom described in the literature. When described, the method of preparation of microcapsules with liquid cores usually involves the preparation of an emulsion and consequent formation of a shell and hardening of the shell around the dispersed droplets, which form a core of the microcapsules. Such methods of preparation, which also include alginate shell formation, are described, for instance in WO 2007/129926 A, US 5 753 264 A and by Ribeiro AJ et al., Int J Pharm. 1999;187(1):115-23. Whereas such methods are suitable for preparation of microcapsules where the core phase and shell phase are not miscible, they are unsuitable for preparation of microcapsules containing SMES as the core, since one cannot prepare a starting emulsion because SMES readily mixes with water and forms a microemulsion. Thus all methods employing starting emulsion with the water as the outer phase (i.e. the vast majority of the methods) are unable to provide distinct droplets or cores, which could be converted to microcapsules. Methods of preparation which include non aqueous outer phase are also in great majority unable to form distinct droplets since SMES readily mixes with the majority of non aqueous solvents and they are not suitable for the preparation of microcapsules with alginate shell, since alginate is not soluble in organic solvents.

A vibrating nozzle method is often used for alginate bead preparation, since it allows a high production rate of uniform sized beads with a mean diameter below 300 µm. The process can be carried out under mild conditions and can easily be scaled up. Because it also allows complete sterility of the process, present day applications are mostly oriented towards cell encapsulation. Microcapsules can be prepared without the formation of a starting emulsion, which is also true for some other methods, like for instance jet cutter, laminar jet or multi-orifice centrifugal processes.

When microspheres or microcapsules are prepared from alginate, a gelling agent is usually used to convert the dissolved alginate (in the form of salts e.g. sodium or potassium alginate) from liquid (sol) to solid (gel) state and thus harden the microspheres/microcapsules. The role of the gelling agent is to immobilize alginate polymer chains an to form a solid structure, which gives physical stability to the formed product. The most common gelling agent for sodium or potassium alginate gelation are divalent cations like Ca²⁺, Ba²⁺ and Zn²⁺ which form water insoluble alginate salts by displacing monovalent ions in the alginate salt (e.g. sodium or potassium ions).

Hitherto, the application of SMES is limited to liquid dosage forms and soft gelatin capsules, which are not always optimal formulations for oral application. An inclusion of such systems in microcapsules can greatly broaden formulation possibilities, most notably allowing the formulation of solid dosage forms.

The need to formulate self-microemulsifying systems or microemulsions into solid dosage forms has long been recognized in the pharmaceutical sciences. Current commercial products only include liquid formulations and semi solid formulations in the form of soft gelatin capsules. The majority of other approaches involve the absorption of self-microemulsifying systems or microemulsions on suitable carriers, e.g. colloidal silica. The formulation of gels, which can be filled into capsules or the formation of homogeneous matrix microparticles, is also reported. Only one case of microcapsules loaded with a self-microemulsifying system is described in the literature.

US 6 280 770 discloses pharmaceutical compositions, which improve the rate and/or extent of absorption of drugs. The pharmaceutical compositions of this document comprise drug-containing microemulsions adsorbed onto solid particles which may be further formulated into solid dosage forms.

US 2007/0009559 A1 discloses free-flowing solid formulations of drugs or pharmaceutical agents, which have a poor aqueous solubility and are obtained by admixing a liquid or gel composition that includes 1 to 30 percent by weight of the drug, 5 to 60 percent by weight of a surfactant, 10 to 40 percent by weight of water; 1 to 20 percent by weight of unsaturated fatty acid ester, 0 to 50 percent by weight of water miscible pharmaceutically acceptable polyol and 1 to 10 percent by weight of phospholipid with a pharmaceutically acceptable suitable solid carrier. Thereafter, the admixture is dried. The free-flowing powder is suitable for being formed into tablets or capsules. The drug or pharmaceutical agent is solubilized in the formulation.

Kim, C-K et al., Pharm Res. 2001 Apr; 18(4):454-9 describes the preparation of solid state self microemulsifying systems either by mixing with an organic solution of different polymers or by addition of alginate and subsequent gelling by dropping into an aqueous solution of CaCl₂. The described procedure does not result in microcapsules as described earlier, but rather in a matrix type system in which self-microemulsifying system or its components are dispersed throughout the carrier polymer. Addition of a gelling agent to self-microemulsifying phase to promote solidification as soon as the core and shell phase are in contact is not described.

Homar M, et al., J Microencapsul. 2007 Feb; 24(1):72-81 describes the preparation of microcapsules with a self-microemulsifying core with drug loading up to 0.07%. This publication describes the incorporation of minute amount of active ingredients into the microcapsules. Addition of a gelling agent to self-microemulsifying (core) phase to promote solidification as soon as the core and shell phase are in contact is not described. However, with the majority of active pharmaceutical ingredients, the described incorporated amount of an active ingredient is too low for manufacturing a suitably sized solid oral preparation.

According to a preferred embodiment of the present invention, microcapsules having a shell and a liquid core incorporating a self-microemulsifying system or a microemulsion are provided, wherein the core comprises a lipophilic substance, at least one surfactant, an active agent and optionally a cosolvent, characterized in that the core further comprises a gelling agent. The gelling agent in the core promotes the solidification of the shell as soon as core and shell phase are in contact, allowing for much higher core entrapment and reduces the loss of core phase and the active pharmaceutical ingredient during preparation, thus allowing form much higher drug loadings, which cannot be obtained by currently known methods. The concentration of the gelling agent must be high enough to ensure proper shell hardening. Preferably the core is saturated with the gelling agent to ensure optimal microcapsule formation.

The lipophilic substance may be selected from, but is not limited to, the group consisting of mono-, di- and triglycerides, including oils, or fatty acids and their esters and esters of propylene glycol or other polyols. The fatty acids and esters are used as such, or where they form part of a glyceride, they may have a short chain, a medium chain or a long chain. The substance may be of vegetable or animal origin, synthetic or semisynthetic. The oils include, but are not limited to natural oils, such as cottonseed oil, soybean oil, sunflower oil; canola oil; Captex® (various grades); Miglyol®; and Myvacet®, as well as mixtures from two or more of these substances.

The surfactant may be selected from, but is not limited to, the group consisting of gelatin, lecithin (phosphatides), gum acacia, cholesterol, tragacanth, polyoxyethylene alkyl ethers, polyoxyethylene castor oil derivatives, polyoxyethylene sorbitan fatty acid esters, sorbitan fatty acid esters, polyethylene glycols, polyoxyethylene stearates, mono and diglycerides, colloidal silicon dioxide, sodium dodecylsulfate, magnesium aluminum silicate, triethanolamine, polyvinyl alcohol, and polyvinylpyrrolidene (PVP), stearic acid, calcium stearate, glycerol monostearate, cetostearyl alcohol, cetomacrogol emulsifying wax, short and medium chain alcohols, or various grades of the following commercial products: Labrafac® (Medium-chain triglycerides); Labrafil® (natural oil - polyoxyethylene esters); Labrasol® (caprylic/capric glycerides); Plurololeique® (Polyglyceryl-6 dioleate), as well as mixtures from two or more of these substances.

The core is surrounded by a shell, which is preferably formed from polymeric material which may comprise a substance selected from, but not limited to, the group consisting of alginate of various grades and chitosan of various grades.

The gelling agent is selected according to the polymer used for shell formation. It may be selected from, but is not limited to, the group consisting of various divalent (Ca²⁺, Zn²⁺, Ba²⁺, Cu²⁺) and trivalent (Al³⁺) ions used for the crosslinking of alginate or in the case of chitosan, from the group consisting of tripolyphosphate, citric acid and glutaraldehyde, as well as mixtures from two or more of these ions or substances. Preferably, the core is saturated with the gelling agent. In a particular preferred embodiment the shell is formed from alginate and the gelling agent are Ca²⁺ ions.

The cosolvent may be selected from, but is not limited to, the group consisting of triacetin (1,2,3-propanetriyl triacetate or glyceryl triacetate) or other polyol esters of fatty acids, trialkyl citrate esters, propylene carbonate, dimethylisosorbide, ethyl lactate, N-methyl pyrrolidones, Transcutol® (diethylene glycol monoethyl ether), glycofurol, peppermint oil, 1,2-propylene glycol, ethanol, and polyethylene glycols, as well as mixtures from two or more of these substances.

The active agent may be selected from, but is not limited to, the group of drug classes consisting of analgesics/antipyretics; antibiotics; antidepressants; antidiabetics; antifungal agents; antihypertensive agents; anti-inflammatories; antineoplastics; antianxiety agents; antimigraine agents; sedatives/hypnotics; antianginal agents; antimanic agents; antiarrhythmics; antiarthritic agents; antigout agents; antifibrinolytic; antiplatelet agents; anticonvulsants; antiparkinson agents; antihistamines/antipruritics; agents useful for calcium regulation; antibacterial agents; antiviral agents; antimicrobials; anti-infectives; bronchodilators; hormones; hypoglycemic agents; hypolipidemic agents; proteins; nucleic acids; agents useful for erythropoiesis stimulation; antiulcer/antireflux agents; antinauseants/antiemetics; oil-soluble vitamins, as well as suitable mixtures from two or more of these substances.

The ratio of the active ingredient, the lipophilic substance, the surfactant(s), the gelling agent and the cosolvent (if present) depends upon the efficiency of emulsification, the efficiency of the gelling agent and the solubility, and the solubility depends on the dose per unit that is desired.

Preferably, based on the total mass of the core, the components for a self-emulsifying core may be in the following ranges (weight percent):
1-50% active ingredient;
1-80% lipophilic substance;
5-90% surfactant and co-surfactant;
0.01-20% gelling agent, preferably 0.1-10%, more preferably 1-5%;
0-60% cosolvent.

According to a further preferred embodiment of the present invention, a method for producing microcapsules is provided, comprising the steps of preparing a core by mixing a lipophilic substance and at least one surfactant; adding a gelling agent to the core; incorporating an active agent in the core; preparing a shell forming phase; forming microcapsules having a core and a shell; and incubating the formed microcapsules in a gelling solution.

Preferably, the method further comprises a step of coating the microcapsules with a substance selected from, but not limited to, the group consisting of chitosan, carboxymethylcellulose sodium, methylcellulose, hydroxyethylcellulose, hydroxypropylcellulose, hydroxypropylmethylcellulose phthalate, polyvinylpyrrolidene (PVP) and polymethacrylates (e.g. various grades of Eudragit®), as well as mixtures from two or more of these substances.

Preferably, in the method, the formation of microcapsules is performed by using a vibrating nozzle device.

For the formation of microcapsules, the flow rates of both the core and the shell forming phase are important for the final physico-chemical properties of the microcapsules and the dose per unit that is desired. The ratio between the phases depends on the characteristics of both the core and the shell forming phase and the gelling ability and the intensity of the gelling agent. Generally, the ratio of the flow rates of the core forming phase and the shell forming phase is in the interval between 1:0.01 and 1:100, preferably 1:0.1 and 1:50, more preferably 1:1 and 1:10.

In order to increase the contact between the gelling agent in the core phase and the shell phase before the final microcapsule is incubated in the gelling solution, the flow speed of both phases is decreased as much as possible. The actual flow rates depend on the characteristics of both the core forming phase and the shell forming phase, gelling ability and intensity of the gelling agent and the diameter of the nozzle. Furthermore, the distance traveled by the formed microcapsule before it reaches the incubation solution can be increased.

According to a further preferred embodiment of the present invention, a pharmaceutical formulation, comprising microcapsules as defined above is provided. Preferably, the pharmaceutical formulation is a solid dosage form, more preferably a capsule or a tablet.

The technical concept of the invention enables the incorporation of self microemulsifying systems or microemulsions into microcapsules with a liquid core, which can be further formulated into solid dosage forms. The concept significantly increases the amount of active ingredients that can be incorporated into liquid core microcapsules and thus makes the manufacturing of a suitably sized solid oral preparation feasible.

In a particularly preferred embodiment, microcapsules with a liquid self-microemulsifying system in the core, which are suitable for further use in the production of solid oral dosage forms were prepared according to the following method:
At first, a self microemulsifying system comprising preferably Miglyol 812 as a lipophilic substance, and preferably a mixture of Labrasol as surfactant and co-surfactant and Plurol oleique was prepared by mixing the surfactants and lipophilic (oily) phase to form a clear, homogeneous mixture. Further, the resulting SMES was saturated with a gelling agent, preferably CaCl₂, and was used as the core phase. An active ingredient, e.g. celecoxib, was incorporated into the core phase prior to microcapsule formation. A sodium alginate solution was prepared by mixing sodium alginate with water until all sodium alginate has been dissolved. Further on, lactose and sodium chloride were added to sodium alginate solution and the resulting solution was used as the shell forming phase. Core and shell forming phases were fed to an Inotech IE-50 R encapsulator with binary nozzle with the help of syringes or a pressurized vessel. The flow velocities of both phases were optimized to yield round microcapsules with a centrally positioned core. The amplitude and the frequency of the membrane were adjusted to achieve best microcapsule sphericity. The resulting microcapsules were incubated in the gelling solution and further coated with a chitosan. The final step consisted of the drying of microcapsules in a fluid bed system.

### Examples

The present invention is further illustrated but in no way limited by the following examples.

### EXAMPLE 1

The first step for the production of microcapsules involves the preparation of a self-microemulsifying system. Labrasol® (PEG-8 caprylic/capric glycerides) and Plurol oleique® (Polyglyceryl-6 dioleate) were mixed in a 4:1 ratio with a magnetic stirrer being used. Miglyol 812® (Caprylic/Capric Triglyceride) (20 % w/w) was added to the mixture, resulting in a homogeneous SMES. The system was saturated with CaCl₂ and subsequently centrifuged to yield a clear solution of SMES saturated with the salt. An alginate solution used for the formation of the shell was prepared by mixing sodium alginate (1,5% w/w), lactose (5% w/w) and sodium chloride (1% w/w) with purified water.

An Inotech IE-50 R encapsulator (Inotech, Swiss) equipped with a 500 µm / 750 µm concentric nozzle, a 50 ml syringe and an air pressure solution delivery system was used to prepare microcapsules with a self-microemulsifying core. The above self-microemulsifying system saturated with CaCl₂ was mixed with celecoxib and used as the core forming phase. Microcapsules were produced at a shell flow rate of 44.6 mg/s. The amplitude of the membrane was constant throughout all experiments and its frequency was set to 3000 Hz. Microcapsules were incubated in 0.5 M CaCl₂ solution for 5 minutes and dried in an Aeromatic Strea 1 fluid bed system. Microcapsules were dried at an inlet air temperature setting of 55 °C until the outlet air temperature reached 50 °C. The volume of fluidizing air was regulated in the range from 80 to 120 m³/h in order to ensure an optimal fluidizing of the microcapsules.

The celecoxib content was calculated as the amount of celecoxib based on the total mass of dried microcapsules. To measure the amount of the active ingredient, dried celecoxib loaded microcapsules were incubated in a medium containing 2% Tween 80 and 1% NaCl for 24 hours. After incubation the remaining capsules were crushed and sonicated for 15 minutes. Samples were filtered through a 0.45 µm cellulose acetate filter (Sartorius, Germany) and analyzed by means of an HPLC system.

The degree of encapsulation of celecoxib was expressed as a percentage of the total amount of celecoxib used for microcapsule preparation.

The specific parameters of the preparation and the characteristics of the produced microcapsules are indicated in Table 1.

**Table 1**

| **Preparation parameters** | |
|---|---|
| Core composition | Labrasol, Plurol oleique, Miglyol 812 |
| Gelling agent added to the core | Solid CaCl₂ |
| Chitosan coating | Not present |
| SMES : celecoxib ratio | 2:1 |
| Core phase flow rate | 7.3 mg/s |
| Shell phase flow rate | 44.6 mg/s |
| Membrane frequency | 3000 Hz |
| Membrane amplitude | Medium |
| | |

| **Characteristics of microcapsules** | |
|---|---|
| Celecoxib content (%) | 17.5 ± 4.5 |
| Degree of encapsulation (%) | 75.3 ± 7.4 |
| Core description | Semi solid celecoxib/SMES mixture |

### EXAMPLE 2

In this Example, microcapsules were prepared substantially following the procedure described in Example 1. Additionally, prior to drying microcapsules were incubated for 5 minutes in a 1 mg/mL chitosan solution. The specific parameters of the preparation and the characteristics of the produced microcapsules are indicated in Table 2.

**Table 2**

| **Preparation parameters** | |
|---|---|
| Core composition | Labrasol, Plurol oleique, Miglyol 812 |
| Gelling agent added to the core | Solid CaCl₂ |
| Chitosan coating | Present |
| SMES : celecoxib ratio | 2:1 |
| Core phase flow rate | 7.3 mg/s |
| Shell phase flow rate | 44.6 mg/s |
| Membrane frequency | 3000 Hz |
| Membrane amplitude | Medium |
| | |

| **Characteristics of microcapsules** | |
|---|---|
| Celecoxib content (%) | 24.4 ± 7.9 |
| Degree of encapsulation (%) | 77.3 ± 6.8 |
| Core description | Semi solid celecoxib/SMES mixture |

### EXAMPLE 3

In this Example, microcapsules were prepared substantially following the procedure described in Example 1. In deviation from Example 1, the core phase was saturated with CaCl₂ by the addition of 1% 6M CaCl₂ solution instead of solid CaCl₂. The specific parameters of the preparation and the characteristics of the produced microcapsules are indicated in Table 3.

**Table 3**

| **Preparation parameters** | |
|---|---|
| Core composition | Labrasol, Plurol oleique, Miglyol 812 |
| Gelling agent added to the core | 6M CaCl₂ aqueous solution |
| Chitosan coating | Not present |
| SMES : celecoxib ratio | 2:1 |
| Core phase flow rate | 7.3 mg/s |
| Shell phase flow rate | 44.6 mg/s |
| Membrane frequency | 3000 Hz |
| Membrane amplitude | Medium |
| | |

| **Characteristics of microcapsules** | |
|---|---|
| Celecoxib content (%) | 26.2 ± 1.9 |
| Degree of encapsulation (%) | 82.3 ± 4.2 |
| Core description | Semi solid celecoxib/SMES mixture |

### EXAMPLE 4

In this Example, microcapsules were prepared substantially following the procedure described in Example 1. The specific parameters of the preparation and the characteristics of the produced microcapsules are indicated in Table 4.

**Table 4**

| **Preparation parameters** | |
|---|---|
| Core composition | Labrasol, Plurol oleique, Miglyol 812 |
| Gelling agent added to the | Solid CaCl₂ |
| core | |
| Chitosan coating | Not present |
| SMES : celecoxib ratio | 4:1 |
| Core phase flow rate | 40.1 mg/s |
| Shell phase flow rate | 44.6 mg/s |
| Membrane frequency | 3000 Hz |
| Membrane amplitude | Medium |
| | |

| **Characteristics of microcapsules** | |
|---|---|
| Celecoxib content (%) | 25.4 ± 2.0 |
| Degree of encapsulation (%) | 59.2 ± 5.8 |
| Core description | Liquid celecoxib/SMES solution |

### EXAMPLE 5

In this Example, microcapsules were prepared substantially following the procedure described in Example 1. In deviation from Example 1, prior to drying microcapsules were additionally incubated for 5 minutes in 1 mg/mL chitosan solution. The specific parameters of the preparation and the characteristics of the produced microcapsules are indicated in Table 5.

**Table 5**

| **Preparation parameters** | |
|---|---|
| Core composition | Labrasol, Plurol oleique, Miglyol 812 |
| Gelling agent added to the core | Solid CaCl₂ |
| Chitosan coating | Present |
| SMES : celecoxib ratio | 4:1 |
| Core phase flow rate | 40.1 mg/s |
| Shell phase flow rate | 44.6 mg/s |
| Membrane frequency | 3000 Hz |
| Membrane amplitude | Medium |
| | |

| **Characteristics of microcapsules** | |
|---|---|
| Celecoxib content (%) | 33.0 ± 3.4 |
| Degree of encapsulation (%) | 62.4 ± 6.3 |
| Core description | Liquid celecoxib/SMES solution |

### COMPARATIVE EXAMPLE

As a comparative example, microcapsules were prepared without the use of a gelling agent in the core phase. The first step for producing microcapsules was similar to the procedure described in the Example 1 without the saturation of the SMES mixture with CaCl₂. An alginate solution used for the formation of the shell was prepared by mixing sodium alginate (1,5% w/w) and lactose (5% w/w) with purified water.

An Inotech IE-50 R encapsulator (Inotech, Swiss) equipped with a 150 µm / 250 µm concentric nozzle, and two 50 ml syringes were used to prepare microcapsules with a self-microemulsifying core. The self-microemulsifying system was mixed with ketoprofen and was used as the core forming phase. The amplitude of the membrane was constant throughout all experiments and its frequency was set to 550 Hz. Microcapsules were incubated in a 0.3 M CaCl₂ solution adjusted to pH 3 for 30 minutes, rinsed with purified water and dried in an Aeromatic Strea 1 fluid bed system. Microcapsules were dried at an inlet air temperature setting of 55 °C until the outlet air temperature reached 47 °C. The volume of fluidizing air was regulated in the range from 80 to 120 m³/h in order to ensure an optimal fluidizing of the microcapsules.

The ketoprofen content was calculated as the amount of ketoprofen with respect to the total mass of dried microcapsules. The produced microcapsules were crushed and incubated in an NaOH solution at pH 9 for 24 hours. After incubation, the mixture was sonicated for 15 minutes, centrifuged at 3000 rpm for 15 minutes, and the supernatant was filtered through a 0.22 µm cellulose acetate filter. The concentration of ketoprofen was determined by absorbance measurement at 260 nm. Microcapsules without ketoprofen, produced under the same operating parameters and treated in the same way, were used to set the baseline to zero. Entrapment efficacy was calculated as the amount of encapsulated ketoprofen relative to the total amount of ketoprofen in the microcapsules, hardening solution and water used for rinsing.

The specific parameters of the preparation and the characteristics of the produced microcapsules are indicated in Table 6.

**Table 6**

| **Preparation parameters** | |
|---|---|
| Core composition | Labrasol, Plurol oleique, Miglyol 812 |
| Gelling agent added to the core | None |
| Chitosan coating | Not present |
| SMES : ketoprofen ratio | 9:1 |
| Core phase flow rate | 0.061 mg/s |
| Shell phase flow rate | 7.290 mg/s |
| Membrane frequency | 550 Hz |
| Membrane amplitude | Medium |

| | |
|---|---|
| | |

| **Characteristics of microcapsules** | |
|---|---|
| Ketoprofen content (%) | 0.0684 ± 0.00344 |
| Degree of encapsulation (%) | 87.49 ± 9.87 |
| Core description | No core could be observed |

As observed by comparison of the content of the active ingredient in the EXAMPLES 1-5 and the COMPARATIVE EXAMPLE, one can conclude that the absence of the gelling agent in the core phase significantly reduces the content of the active ingredient. Furthermore, the amount of active ingredient is reduced below the amount suitable for the preparation of most solid oral dosage forms. The increase in the flow speed of core phase, which would in turn increase the active ingredient content is not possible, since no microcapsules are formed due to intense mixing of the core and shell phase.

## Claims

1. A microcapsule having a shell and a liquid core incorporating a self-microemulsifying system or a microemulsion, wherein the core comprises a lipohilic substance, at least one surfactant, an active agent, and optionally a cosolvent,
**characterized in that** the core further comprises a gelling agent.

2. The microcapsule according to claim 1, wherein the lipohilic substance is selected from the group consisting of mono-, di- and triglycerides, including oils, or fatty acids and their esters and esters of propylene glycol or other polyols, or mixtures from two or more of these substances.

3. The microcapsule according to claim 1, wherein the surfactant is selected from the group consisting of gelatin, lecithin, phosphatides, gum acacia, cholesterol, tragacanth, polyoxyethylene alkyl ethers, polyoxyethylene castor oil derivatives, polyoxyethylene sorbitan fatty acid esters, sorbitan fatty acid esters, polyethylene glycols, polyoxyethylene stearates, mono- and diglycerides, colloidal silicon dioxide, sodium dodecylsulfate, magnesium aluminum silicate, triethanolamine, polyvinyl alcohol, and polyvinylpyrrolidene, stearic acid, calcium stearate, glycerol monostearate, cetostearyl alcohol, cetomacrogol emulsifying wax, short and medium chain alcohols, or mixtures from two or more of these substances.

4. The microcapsule according to claim 1, wherein the cosolvent is selected from the group consisting of 1,2,3-propanetriyl triacetate or glyceryl triacetate or other polyol esters of fatty acids, trialkyl citrate esters, propylene carbonate, dimethylisosorbide, ethyl lactate, N-methyl pyrrolidones, diethylene glycol monoethyl ether, glycofurol, peppermint oil, 1,2-propylene glycol, ethanol, and polyethylene glycols, or mixtures from two or more of these substances.

5. The microcapsule according to claim 1, wherein the core is surrounded by a shell formed from a polymeric material.

6. The microcapsule according to claim 5, wherein the shell is formed from alginate.

7. The microcapsule according to claim 5, wherein the shell is formed from chitosan.

8. The microcapsule according to claim 6, wherein the gelling agent is selected from the group consisting of divalent and trivalent ions, preferably Ca²⁺, 2n²⁺, Ba²⁺, Cu²⁺, or Al³⁺, or mixtures from two or more of these ions.

9. The microcapsule according to claim 7, wherein the gelling agent is selected from the group consisting of tripolyphosphate, citric acid and glutaraldehyde or mixtures from two or more of these substances.

10. The microcapsule according to any one of the preceding claims, wherein the core is saturated with the gelling agent.

11. The microcapsule according to claim 1, wherein the active agent is selected from drug classes consisting of analgesics/antipyretics; antibiotics; antidepressants; antidiabetics; antifungal agents; antihypertensive agents; anti-inflammatories; antineoplastics; antianxiety agents; antimigraine agents; sedatives/hypnotics; antianginal agents; antimanic agents; antiarrhythmics; antiarthritic agents; antigout agents; antifibrinolytic; antiplatelet agents; anticonvulsants; antiparkinson agents; antihistamines/antipruritics; agents useful for calcium regulation; antibacterial agents; antiviral agents; antimicrobials; anti-infectives; bronchodilators; hormones; hypoglycemic agents; hypolipidemic agents; proteins; nucleic acids; agents useful for erythropoiesis stimulation; antiulcer/antireflux agents; antinauseants/antiemetics; oil-soluble vitamins, or suitable mixtures from two or more of these drugs.

12. The microcapsule according to any one of the preceding claims, wherein the core is coated with a substance selected from the group consisting of chitosan, carboxymethylcellulose sodium, methylcellulose, hydroxyethylcellulose, hydroxypropylcellulose, hydroxypropylmethylcellulose phthalate, polyvinylpyrrolidene and polymethacrylates, or mixtures from two or more of these substances.

13. A method for producing microcapsules according to claims 1 to 12, comprising the steps of
preparing a core phase by mixing a lipophilic substance and at least one surfactant;
adding a gelling agent to the core phase; incorporating an active agent in the core phase; preparing a shell forming phase;
forming microcapsules having a core and a shell; and incubating the formed microcapsules in a gelling solution; and
optionally coating the microcapsules.

14. The method according to claim 13, wherein the formation of microcapsules is performed by using a vibrating nozzle device.

15. A pharmaceutical formulation comprising the microcapsules according to claims 1 to 12.

## Patentansprüche

1. Mikrokapsel, die eine Schale und einen flüssigen Kern aufweist, der ein selbst-mikroemulgierendes System oder eine Mikroemulsion enthält, wobei der Kern eine lipophile Substanz, wenigstens einen oberflächenaktiven Stoff, einen Wirkstoff und optional ein Co-Lösungsmittel enthält,
**dadurch gekennzeichnet, dass** der Kern außerdem ein Geliermittel umfasst.

2. Mikrokapsel nach Anspruch 1, wobei die lipophile Substanz ausgewählt ist aus der Gruppe bestehend aus Mono-, Di- und Triglyceriden, einschließlich Ölen, oder Fettsäuren und deren Estern und Estern von Propylenglykol oder anderen Polyolen, oder Mischungen von zwei oder mehr dieser Substanzen.

3. Mikrokapsel nach Anspruch 1, wobei der oberflächenaktive Stoff ausgewählt ist aus der Gruppe bestehend aus Gelatine, Lecithin, Phosphatiden, Gummiarabikum, Cholesterin, Tragakanth, Polyoxyethylenalkylethern, Polyoxyethylen-Castorölderivaten, Polyoxyethylen-Sorbitanfettsäureestern, Sorbitanfettsäureestern, Polyethylenglycolen, Polyoxyethylenstearaten, Mono- und Diglyceriden, kolloidalem Siliciumdioxid, Natriumdodecylsulfat, Magnesiumaluminiumsilikat, Triethanolamin, Polyvinylalkohol, und Polyvinylpyrroliden, Stearinsäure, Calciumstearat, Glycerolmonostearat, Cetostearylalkohol, Cetomacrogol emulgierendes Wachs, kurz- und mittelkettige Alkohole oder Mischungen von zwei oder mehr dieser Substanzen.

4. Mikrokapsel nach Anspruch 1, wobei das Co-Lösungsmittel ausgewählt ist aus der Gruppe bestehend aus 1,2,3-Propantriyltriacetat oder Glyceryltriacetat oder anderen Polyolestern von Fettsäuren, Trialkylcitratestern, Propylencarbonat, Dimetylisosorbid, Ethyllactat, N-Methylpyrrolidonen, Diethylenglycolmonoethylether, Glycofurol, Pfefferminzöl, 1,2-Propylenglycol, Ethanol und Polyethylenglycolen, oder Mischungen von zwei oder mehr dieser Substanzen.

5. Mikrokapsel nach Anspruch 1, wobei der Kern von einer Schale umgeben ist, die aus einem polymeren Material gebildet ist.

6. Mikrokapsel nach Anspruch 5, wobei die Schale aus Alginat gebildet ist.

7. Mikrokapsel nach Anspruch 5, wobei die Schale aus Chitosan gebildet ist.

8. Mikrokapsel nach Anspruch 6, wobei das Geliermittel ausgewählt ist aus der Gruppe bestehend aus zweiwertigen und dreiwertigen Ionen, bevorzugt Ca²⁺, Zn²⁺, Ba²⁺, Cu²⁺ oder Al³⁺, oder Mischungen von zwei oder mehr dieser Ionen.

9. Mikrokapsel nach Anspruch 7, wobei das Geliermittel ausgewählt ist aus der Gruppe bestehend aus Tripolyphosphat, Citronensäure und Glutaraldehyd, oder Mischungen von zwei oder mehr dieser Substanzen.

10. Mikrokapsel nach einem der vorhergehenden Ansprüche, wobei der Kern mit dem Geliermittel gesättigt ist.

11. Mikrokapsel nach Anspruch 1, wobei der Wirkstoff ausgewählt ist aus Arzneimittelklassen bestehend aus Analgetika/Antipyretika; Antibiotika; Antidepressiva; Antidiabetika; Antimykotika; blutdrucksenkende Mittel; entzündungshemmende Mittel; antineoplastische Mittel; Anxiolytika; Antimigränemittel; Sedativa/Hypnotika; antianginöse Mittel; antimanische Mittel; Antiarrhythmika; antiarthritische Mittel; Antigichtmittel; antifibrinolytische Mittel; Antiplättchenmittel; antikonvulsive Mittel; Antiparkinsonmittel; Antihistamine/Antipruritika; Mittel, die für die Calciumregulation verwendbar sind; antibakterielle Mittel; antivirale Mittel; antimikrobielle Mittel; Antiinfektiva; Bronchodilatatoren; Hormone; Hypoglykämika; hyperlipoproteinämische Mittel; Proteine; Nukleinsäuren; Mittel, die für die Erythropoese-Stimulation verwendbar sind; Antiulkus-/Antirefluxmittel; Mittel gegen Übelkeit/Antiemetika; öllösliche Vitamine oder geeignete Mischungen von zwei oder mehr dieser Arzneimittel.

12. Mikrokapsel nach einem der vorhergehenden Ansprüche, wobei der Kern mit einer Substanz beschichtet ist, die ausgewählt ist aus der Gruppe bestehend aus Chitosan, Carboxymethylcellulosenatrium, Methylcellulose, Hydroxyethylcellulose, Hydroxypropylcellulose, Hydroxypropylmethylcellulosephthalat, Polyvinylpyrroliden und Polymethacrylaten, oder Mischungen von zwei oder mehr dieser Substanzen.

13. Verfahren für die Herstellung von Mikrokapseln nach Ansprüchen 1 bis 12, umfassend die Schritte von
Zubereiten einer Kernphase durch Mischen einer lipophilen Substanz und wenigstens eines oberflächenaktiven Stoffs;
Zugeben eines Geliermittels zu der Kernphase;
Einbringen eines Wirkstoffs in die Kernphase;
Zubereiten einer schalenbildenden Phase;
Bilden von Mikrokapseln mit einem Kern und einer Schale; und Inkubieren der gebildeten Mikrokapseln in einer Gelierlösung; und optional Beschichten der Mikrokapseln.

14. Verfahren nach Anspruch 13, wobei die Bildung von Mikrokapseln durch Verwendung einer vibrierenden Düsenvorrichtung erfolgt.

15. Pharmazeutische Formulierung, die die Mikrokapseln gemäß Ansprüchen 1 bis 12 umfasst.

## Revendications

1. Microcapsule ayant une enveloppe et un noyau liquide incorporant un système auto-microémulsionnant ou une microémulsion, dans laquelle le noyau comprend une substance lipohile, au moins un tensioactif, un principe actif et éventuellement un cosolvant,
**caractérisée en ce que** le noyau comprend en outre un agent gélifiant.

2. Microcapsule selon la revendication 1, dans laquelle la substance lipophile est choisie dans le groupe constitué par les mono-, di- et triglycérides, y compris les huiles, ou les acides gras et leurs esters et esters de propylène glycol ou d'autres polyols, ou des mélanges de deux ou plus de ces substances.

3. Microcapsule selon la revendication 1, dans laquelle le tensioactif est choisi dans le groupe constitué par la gélatine, la lécithine, les phosphatides, la gomme arabique, le cholestérol, la gomme adragante, les éthers d'alkyle et de polyoxyéthylène, les dérivés d'huile de ricin polyoxyéthylénés, les esters d'acide gras et de sorbitan polyoxyéthylénés, les esters d'acide gras et de sorbitan, les glycols polyoxyéthylénés, les stéarates de polyoxyéthylène, les mono- et diglycérides, le dioxyde de silicium colloïdal, le dodécylsulfate de sodium, le silicate de magnésium et d'aluminium, la triéthanolamine, l'alcool polyvinylique et la polyvinylpyrrolidone, l'acide stéarique, le stéarate de calcium, le monostéarate de glycérol, l'alcool cétostéarylique, une cire émulsionnante cétomacrogol, les alcools à chaîne courte et moyenne, ou des mélanges de deux ou plus de ces substances.

4. Microcapsule selon la revendication 1, dans laquelle le co-solvant est choisi dans le groupe constitué par le triacétate de 1,2,3-propanetriyle ou le triacétate de glycéryle ou d'autres esters de polyol d'acides gras, les esters citrates de trialkyle, le carbonate de propylène, le diméthylisosorbide, le lactate d'éthyle, les N-méthylpyrrolidones, l'éther monoéthylique de diéthylèneglycol, le glycofurol, l'essence de menthe poivrée, le 1,2-propylèneglycol, l'éthanol et les polyéthylène glycols, ou des mélanges de deux ou plus de ces substances.

5. Microcapsule selon la revendication 1, dans laquelle le noyau est entouré par une enveloppe formée d'un matériau polymère.

6. Microcapsule selon la revendication 5, dans laquelle l'enveloppe est formée d'alginate.

7. Microcapsule selon la revendication 5, dans laquelle l'enveloppe est formée de chitosane.

8. Microcapsule selon la revendication 6, dans laquelle l'agent gélifiant est choisi dans le groupe constitué par des ions divalents et trivalents, de préférence le Ca²⁺, le Zn²⁺, le Ba²⁺, le Cu²⁺ ou l'Al³⁺, ou des mélanges de deux ou plus de ces ions.

9. Microcapsule selon la revendication 7, dans laquelle l'agent gélifiant est choisi dans le groupe constitué par le tripolyphosphate, l'acide citrique et le glutaraldéhyde ou des mélanges de deux ou plus de ces substances.

10. Microcapsule selon l'une quelconque des revendications précédentes, dans laquelle le noyau est saturé avec l'agent gélifiant.

11. Microcapsule selon la revendication 1, dans laquelle le principe actif est choisi dans les classes de médicaments constituées par les analgésiques/antipyrétiques ; les antibiotiques ; les antidépresseurs ; les antidiabétiques ; les agents antifongiques ; les agents antihypertenseurs ; les anti-inflammatoires ; les antinéoplasiques ; les agents anxiolytiques ; les agents antimigraineux ; les sédatifs/hypnotiques ; les agents antiangoreux ; les agents anti-manie ; les antiarythmiques ; les agents antiarythmiques ; les agents antigoutteux ; les antifibrinolytiques ; les agents antiagrégants plaquettaires ; les anticonvulsivants ; les agents antiparkinsoniens ; les antihistaminiques/antiprurigineux ; les agents utiles pour la régulation du calcium ; les agents antibactériens ; les agents antiviraux ; les antimicrobiens ; les anti-infectieux ; les bronchodilatateurs ; les hormones ; les hypoglycémiants ; les agents hypolipidémiants ; les protéines ; les acides nucléiques ; les agents utiles pour la stimulation de l'érythropoïèse ; les agents anti-ulcéreux/anti-reflux ; les antinauséeux/antiémétiques ; les vitamines solubles dans l'huile, ou des mélanges appropriés de deux ou plus de ces médicaments.

12. Microcapsule selon l'une quelconque des revendications précédentes, dans laquelle le noyau est enrobé d'une substance choisie dans le groupe constitué par le chitosane, la carboxyméthylcellulose sodique, la méthylcellulose, l'hydroxyéthylcellulose, l'hydroxypropylcellulose, le phtalate d'hydroxypropylméthylcellulose, la polyvinylpyrrolidone et les polyméthacrylates ou des mélanges de deux ou plus de ces substances.

13. Procédé de production de microcapsules selon les revendications 1 à 12, comprenant les étapes de
préparation d'une phase de noyau par mélange d'une substance lipophile et d'au moins un tensioactif ;
ajout d'un agent gélifiant à la phase de noyau ; incorporation d'un principe actif dans la phase de noyau ;
préparation d'une phase de formation d'enveloppe ; formation de microcapsules ayant un noyau et une enveloppe ; et
incubation des microcapsules formées dans une solution de gélification ; et
enrobage éventuel des microcapsules.

14. Procédé selon la revendication 13, dans lequel la formation de microcapsules est réalisée au moyen d'un dispositif à buse vibrante.

15. Formulation pharmaceutique comprenant les microcapsules selon les revendications 1 à 12.
